# EUROPEAN PATENT SPECIFICATION

(11) **EP 3 565 526 B1**
(45) Date of publication and mention of the grant of the patent: **17.04.2024**
(21) Application number: 18701853.6
(22) Date of filing: 05.01.2018
(51) Int. Cl.: A61K 31/4164, A61K 31/4168, A61P 25/02, A61K 9/00, A61K 47/32, A61K 47/38, A61K 47/10

(54) **TOPICAL DETOMIDINE FORMULATIONS**
TOPISCHE DETOMIDIN-FORMULIERUNGEN
FORMULATIONS TOPIQUES DE DÉTOMIDINE

(30) Priority: 06.01.2017 US 201762443174 P
(43) Date of publication of application: 13.11.2019
(73) Proprietor: Clexio Biosciences Ltd., Jerusalem (IL)
(72) Inventor: TSIPORI, Omer, 4283500 Shaar Efraim (IL); ZAMANSKY, Mark, 5221436 Ramat Gan (IL)
(74) Representative: D Young & Co LLP
(86) International application number: PCT/US2018/012579
(87) International publication number: WO 2018/129313

(56) References cited:
- WO-A1-2004/052347
- WO-A1-2006/048501
- YING JIN ET AL: "Ocular Hypotensive Effects of Medetomidine and Its Analogs", JOURNAL OF OCULAR PHARMACOLOGY AND THERAPEUTICS., vol. 7, no. 4, 1 January 1991 (1991-01-01) , pages 285-296, XP055460804, US ISSN: 1080-7683, DOI: 10.1089/jop.1991.7.285

## Description

### TECHNICAL FIELD

The present disclosure pertains to formulations that contain detomidine as defined by the claims and the compositions for use in treating pain using such formulations.

### BACKGROUND

Pain is the most common symptom accompanying, to some degree, almost every medical condition that humans experience. When it is severe enough, it interferes with a patient's ability to function and with quality of life. A common form of pain is that which is associated with peripheral neuropathy, a condition that develops as a result of damage to the peripheral nervous system.

Symptoms of peripheral neuropathy can range from numbness or tingling, to pricking sensations (paresthesia), or muscle weakness. Areas of the body may become abnormally sensitive leading to an exaggeratedly intense or distorted experience of touch (allodynia). When symptoms are severe, they may include burning pain, muscle wasting, paralysis, or organ or gland dysfunction.

There are numerous forms of peripheral neuropathy, and such forms can follow different patterns. Symptoms can be acute or chronic, and can occur over a period of days, weeks, or years. Acute neuropathies, which include Guillain-Barré syndrome, include symptoms that appear suddenly, progress rapidly, and resolve slowly as damaged nerves heal. Symptoms associated with chronic neuropathies can include a subtle start and slow progression, and periods of relief followed by relapse can occur. Other individuals may experience symptoms that reach a certain level of severity and then stay the same for extended periods of time, and still other chronic neuropathies worsen over time.

Diabetic neuropathy represents one of the most common forms of peripheral neuropathy. In this form, nerve damage is progressive: pain and numbness can first occur in both feet, followed by a gradual progression up both legs. Later, the fingers, hands, and arms may become affected. Another form of peripheral neuropathy is postherpetic neuralgia, which is a complication of shingles, which is in turn caused by the chickenpox (*herpes zoster*) virus. Postherpetic neuralgia affects nerve fibers and skin and can cause burning pain that persists for long periods following disappearance of the rash and blisters of shingles.

Although it is generally difficult to control, various strategies have been deployed for treating neuropathic pain. Over-the-counter analgesics such as nonsteroidal antiinflammatory drugs (NSAIDs) can be used to treat mild pain. Medications that are used for chronic neuropathic pain fall under several classes of drugs: antidepressants, anticonvulsant medications, antiarrythmic medications, and narcotic agents. Antidepressants (such as tricyclic antidepressants such as amitriptyline or newer serotonin-norepinephrine reuptake inhibitors such as duloxetine hydrochloride or venlafaxine) and anticonvulsant medications (such as gabapentin, pregabalin, topiramate, and carbamazepine, although other medications used for treating epilepsy) presently represent the most effective types of medications for controlling neuropathic pain. Mexiletine is an antiarrythmic medication that may also be used for treatment of chronic painful neuropathies. Narcotic agents have been prescribed for pain that does not respond to any of the preceding medications. However, narcotic medications can lead to dependence and addiction, and there use is therefore limited to situations in which other treatments have failed.

Certain topically administered medications have been used for addressing neuropathic pain. Lidocaine, an anesthetic agent, and capsaicin, which modifies peripheral pain receptors, represent two prominent examples. Topical agents are generally most appropriate for localized chronic pain, such as that deriving from *herpes zoster* neuralgia (shingles). WO2006/048501 discloses Detomidine (1 wt%) transmucosal preparations, with a pH value of 5-7, the compositions comprising components like glycerol as solubilizers and buffers.

A need persists for additional strategies for alleviating pain associated with a peripheral neuropathy and other conditions imposing acute or chronic effects.

### SUMMARY

The present disclosure provides topical formulations comprising about 0.001 to about 3 wt% detomidine or a salt thereof; and, a carrier that is suitable for topical administration to a subject's skin, wherein the carrier comprises a water-miscible solubilizer that is present in an amount of 0.1-10% by weight of the formulation as defined by the claims; wherein the formulation has a pH of 4.5 to 9, and, wherein the formulation provides prolonged, substantially non-systemic treatment for pain. Such formulations can form a depot of the detomidine or salt thereof in the subject's skin following topical application.

The present disclosure also pertains to compositions for use in providing prolonged, non-systemic treatment for pain in a subject in need thereof comprising topically administering to the subject a formulation according to any of the preceding types.

Also disclosed are methods for forming a subcutaneous depot of detomidine or a salt thereof in a subject comprising topically administering to the subject a formulation according to the above or otherwise disclosed herein, wherein the detomidine or salt thereof is released from the depot into the subject in order to provide prolonged, non-systemic treatment of pain in the subject

The present disclosure also provides topical formulations comprising about 0.001 to about 3 wt% detomidine or a salt thereof; and, a carrier suitable for topical application to the skin of a subject, wherein the formulation is effective to provide relief from pain in the subject while producing a maximum blood plasma concentration of no more than 1600 pg/mL after four days of twice daily administration of the formulation.

Also provided are methods for providing treatment for pain in a subject in need thereof comprising topically administering to the subject a formulation according to any one of the preceding types.

Additional embodiments will be readily apparent on the basis of the disclosure herein.

### BRIEF DESCRIPTION OF THE DRAWINGS

Figures 1-15B and 19-21 all relate to comparative examples.
FIG. 1 depicts blood plasma concentration curves for all tested formulations pursuant to an evaluation of inventive formulations for *in vitro* permeability and pharmacokinetics using a minipig *in vivo* model.
FIG. 2A, 2B, and 2C provide the results of an analysis of pharmacokinetic parameters resulting from testing of inventive formulations.
FIG. 3A and FIG. 3B show the results of an evaluation of the degree of accumulation of detomidine HCl in epidermis and dermis, respectively, following application of inventive formulations, as expressed in terms of ng/cm².
FIGS. 4A and FIG. 4B show the results of an evaluation of the degree of accumulation of detomidine HCl in epidermis and dermis, respectively, as expressed in terms of percentage of applied dose.
FIG. 5 shows the von Frey response in pigs with respect to each of several tested formulations described in Example 2 as measured during the first few hours following topical application immediately after surgery.
FIG. 6 shows the von Frey response in pigs with respect to each of the tested formulations described in Example 2 as measured one hour following each morning topical application over four days.
FIG. 7 shows the von Frey response in pigs with respect to each of the tested formulations described in Example 2 as measured about 15-17 hours following the preceding evening's dose, and before the first dose of the current day, over four days.
FIG. 8 shows plasma concentrations that were measured with respect to test formulations over time following a first dose on Day 0 and prior to the first dose on each of Days 1-4, pursuant to pharmacokinetic evaluation of inventive detomidine formulations in an *in vivo* minipig model.
FIG. 9 shows plasma concentrations that were measured with respect to test formulations over time following a final dose on the fourth day of a pharmacokinetic evaluation of inventive detomidine formulations in an *in vivo* minipig model.
FIG. 10 shows the results of an assessment of the degree of accumulation of detomidine over time in the dermis resulting from application of test formulations according to the present disclosure.
FIG. 11 shows the results of an assessment of the degree of accumulation of detomidine over time in the epidermis resulting from application of test formulations according to the present disclosure.
FIG. 12 shows the results of an assessment of the degree of accumulation of detomidine over time in the stratum corneum, strips 1-10, resulting from application of test formulations according to the present disclosure.
FIG. 13 shows the results of an assessment of the degree of accumulation of detomidine over time in the stratum corneum, strips 11-20, resulting from application of test formulations according to the present disclosure.
FIG. 14 shows the results of an assessment of heart rate of the test pigs over time following administration of test formulations according to the present disclosure.
FIG. 15A shows the von Frey response in pigs with respect to formulations described in Example 4, i.e., a formulation containing 0.1% clonidine, Naropin^{®} (ropivacaine HCl), and a placebo formulation, as measured following topical application immediately after surgery. FIG. 15B shows the von Frey response in pigs with respect to each of these as measured one hour after each morning topical application over five days.
FIG. 16A provides a comparison among the von Frey responses with respect to formulations described in Example 4, i.e., a formulation containing 0.1% detomidine, Naropin^{®} (ropivacaine HCl), and a placebo formulation, as measured following topical application immediately after surgery. FIG. 16B shows the von Frey response with respect to each of these as measured one hour after each morning topical application over five days.
FIG. 17A provides a comparison among the von Frey responses with respect to formulations described in Example 4, i.e., experimental detomidine formulations, a placebo formulation, an injection of Naropin^{®} (ropivacaine HCl) , and a 0.1% clonidine formulation, as measured following topical application immediately after surgery. FIG. 17B shows the von Frey response with respect to each of these formulations as measured one hour after each morning topical application over five days.
FIG. 18 provides a comparison of the von Frey responses among experimental formulations over all days of the relevant study.
FIG. 19 shows the von Frey response in pigs with respect to each of several experimental formulations described in Example 5 as measured during the first few hours following topical application immediately after surgery.
FIG. 20 shows the von Frey response in pigs with respect to each of the experimental formulations described in Example 5 as measured one hour following each morning topical application over five days.
FIG. 21 shows the von Frey response in pigs with respect to each of the experimental formulations described in Example 5 as measured about 15-17 hours following the preceding evening's dose, and one hour before the first dose of the current day, over five days.

### DETAILED DESCRIPTION OF ILLUSTRATIVE EMBODIMENTS

The present inventions may be understood more readily by reference to the following detailed description taken in connection with the accompanying figures and examples, which form a part of this disclosure. It is to be understood that these inventions are not limited to the specific products, methods, conditions or parameters described and/or shown herein, and that the terminology used herein is for the purpose of describing particular embodiments by way of example only and is not intended to be limiting of the claimed inventions.

As employed above and throughout the disclosure, the following terms and abbreviations, unless otherwise indicated, shall be understood to have the following meanings.

In the present disclosure the singular forms "a," "an," and "the" include the plural reference, and reference to a particular numerical value includes at least that particular value, unless the context clearly indicates otherwise. Thus, for example, a reference to "a particle" is a reference to one or more of such particles and equivalents thereof known to those skilled in the art, and so forth. Furthermore, when indicating that a certain element "may be" X, Y, or Z, it is not intended by such usage to exclude in all instances other choices for the element.

When values are expressed as approximations, by use of the antecedent "about," it will be understood that the particular value forms another embodiment. As used herein, "about X" (where X is a numerical value) preferably refers to ±10% of the recited value, inclusive. For example, the phrase "about 8" preferably refers to a value of 7.2 to 8.8, inclusive; as another example, the phrase "about 8%" preferably refers to a value of 7.2% to 8.8%, inclusive. Where present, all ranges are inclusive and combinable. For example, when a range of "1 to 5" is recited, the recited range should be construed as optionally including ranges "1 to 4", "1 to 3", "1-2", "1-2 & 4-5", "1-3 & 5", and the like. In addition, when a list of alternatives is positively provided, such a listing can also include embodiments where any of the alternatives may be excluded. For example, when a range of "1 to 5" is described, such a description can support situations whereby any of 1, 2, 3, 4, or 5 are excluded; thus, a recitation of "1 to 5" may support "1 and 3-5, but not 2", or simply "wherein 2 is not included." The phrase "at least about x" is intended to embrace both "about x" and "at least x".

The present disclosure relates, *inter alia,* to topical formulations comprising about 0.001 to about 3 wt% detomidine or a salt thereof, as defined by the claims and compositions for use in treating pain by topical administration of such formulations. As noted above, pain is perhaps the most common symptom accompanying nearly every medical condition that a human can experience, and certain forms of pain, including those deriving from peripheral neuropathy, remain difficult to treat. The present inventors have developed new topical formulations containing detomidine or a salt thereof that provide relief from pain over an extended period of time. While not intending to be bound to any particular theory of operation, in certain instances, this beneficial effect may result at least partially from the fact that the formulations induce the formation of a depot of the detomidine or salt thereof in the subject's skin, thereby permitting a prolonged effect by release of the drug from the depot over time, and minimizing or avoiding systemic influence of the drug. Although the present formulations can provide treatment of numerous forms of localized pain, of particular benefit is the ability of the formulations to treat pain deriving from a peripheral neuropathy. These and other advantages that are conferred by the present formulations and methods using such formulations are described more fully herein.

Detomidine is a synthetic alpha-2 adrenoreceptor agonist with sedative and analgesic properties. It is presently sold by prescription under the trade name DORMOSEDAN^{®} (Zoetis Services LLC, Parsippany, NJ) as a sedative and anesthetic premedication in connection with a variety of minor surgical and diagnostic procedures on horses and other large animals. It is commonly combined with butorphanol in order to increase the degree of analgesia and depth of sedation, and may also be used with ketamine for intravenous anesthesia of short duration. The route of administration of DORMOSEDAN^{®} injection is typically intramuscular or intravenous, but the drug is also available as a gel (DORMOSEDAN GEL^{®}) that may be administered by the sublingual route.

As described above, the present disclosure pertains to topical formulations comprising detomidine or a salt thereof that are effective for treating pain in a human subject as defined by the claims.

While not intending to be bound by any particular theory of operation, the inventive formulations surprisingly can form a depot of the detomidine or salt thereof following topical application to a subject's skin. As used herein, a "depot" represents an accumulation or a deposit of drug in a localized mass, from which the drug is gradually released to the surrounding tissue, thereby providing a prolonged, non-systemic effect for the treatment of pain. The present formulations can result in the formation of a depot of the detomidine or salt thereof in the subcutaneous tissue (hypodermis), the epidermis, or the dermis of a subject's skin. The formulations limit systemic exposure to the active agent and can achieve target engagement at the skin nociceptors in the epidermal skin layer. In certain embodiments, the depot includes crystals of the detomidine or salt thereof. Such crystals form within the skin, including one or more of its layers, of the subject on which the formulation is topically applied, *i.e.,* the crystals form following delivery of the detomidine or salt thereof into the subject's skin.

In accordance with these and other features, provided are topical formulations comprising about 0.001 to about 3 wt% detomidine or a salt thereof; and, a carrier that is suitable for topical administration to a subject's skin, wherein the carrier optionally comprises a water-miscible solubilizer that is present in an amount of 0.1-10% by weight of the formulation; wherein the formulation has a pH of 4.5 to 9, and, wherein the formulation provides prolonged, substantially non-systemic treatment for pain, as defined by the claims. Topical administration of the formulations to a subject can be effective to induce formation of a depot of detomidine or a salt thereof in the subject's skin.

The formulations contain about 0.001 to about 3 wt% detomidine or a salt thereof. For example, the formulations may include about 0.005 to about 3, about 0.003 to about 3, about 0.008 to 3, about 0.01 to about 3, about 0.01 to about 2, about 0.01 to about 1.5, about 0.01 to about 1, 0.033 to about 1, 0.033 to about 0.33, about 0.05 to about 3, about 0.1 to about 3, about 0.1 to about 2.5, about 0.1 to about 2, about 0.1 to about 1.5, about 0.1 to about 1, about 0.33 to about 1, about 0.5 to about 1, or about 0.5 to about 0.75 wt% detomidine or salt thereof, or about 0.001, about 0.002, about 0.003, about 0.005, about 0.007, about 0.008, about 0.009, about 0.01, about 0.03, about 0.05, about 0.075, about 0.08, about 0.09, about 0.1, about 0.2, about 0.3, about 0.33, about 0.5, about 0.7, about 0.75, about 0.8, about 1.0, about 1.2, about 1.25, about 1.33, about 1.4, about 1.5, about 1.6, about 1.7, about 1.8, about 1.9, about 2.0, about 2.1, about 2.2, about 2.3, about 2.33, about 2.4, about 2.5, about 2.6, about 2.7, about 2.8, about 2.9, or about 3 wt% of detomidine or salt thereof, wherein "about" is relating to +/- 10% of the recited value.

The detomidine may be present in the formulations in the free base form or as a salt. Those of ordinary skill in the art can readily identify exemplary pharmaceutically acceptable salt forms of detomidine. Suitable pharmaceutically acceptable salts of detomidine include detomidine bitartrate, detomidine bitartrate hydrate, detomidine hydrochloride, detomidine p-toluenesulfonate, detomidine phosphate, detomidine thiosemicarbazone, detomidine sulfate, detomidine trifluoroacetate, detomidine hemipentahydrate, detomidine bitartrate hemipentahydrate, detomidine pentafluoropropionate, detomidine p-nitrophenylhydrazone, detomidine o-methyloxime, detomidine semicarbazone, detomidine hydrobromide, detomidine mucate, detomidine oleate, detomidine phosphate dibasic, detomidine phosphate monobasic, detomidine inorganic salt, detomidine organic salt, detomidine acetate trihydrate, detomidine bis(heptafluorobutyrate), detomidine bis(methylcarbamate), detomidine bis(pentafluoropropionate), detomidine bis(pyridine carboxylate), detomidine bis(trifluoroacetate), detomidine chlorhydrate, and detomidine sulfate pentahydrate. In certain embodiments of the presently disclosed dosage forms, the detomidine is present as the hydrochloride salt.

The formulations as defined by the claims, also include a carrier that is suitable for topical administration to a subject's skin. The carrier includes a solubilizer as defined by the claims and may include, for example, a buffer. As described below, the formulation also comprises a gelling agent as defined by the claims and may also include one or more additional components in order to produce the topical form, such as preservatives, antioxidants, permeation enhancers, emulsifying agents, emollients, or humectants.

Accordingly the formulations includes 0.1-10% by weight of one or more solubilizers, such as a water-miscible solubilizer. The total amount of water-miscible solubilizer may be, for example, about 0.1 to about 10, or about 0.1 to about 5% by weight, or about 0.1, about 0.2, about 0.3, about 0.4, about 0.5, about 0.6, about 0.7, about 0.8, about 0.9, about 1.0, about 1.1, about 1.2, about 1.3, about 1.4, about 1.5, about 1.6, about 1.7, about 1.8, about 1.9, about 2.0, about 2.1, about 2.2, about 2.3, about 2.4, about 2.5, about 2.6, about 2.7, about 2.8, about 2.9, about 3.0, about 3.1, about 3.2, about 3.3, about 3.4, about 3.5, about 3.6, about 3.7, about 3.8, about 3.9, about 4, about 5, about 6, about 7, about 8, about 9, about 10, about 12, about 14, about 16, about 18, about 20, about 22, about 25, about 27, about 30, about 32, about 35, about 37, or about 40 wt% in the present formulations. In some embodiments, the total amount of the solubilizer, e.g., water-miscible solubilizer, is not more than two times, not more than three times, or not more than four times the amount of the detomidine or salt thereof in the formulation.

The water-miscible solubilizers for the present formulations include alcohols, such as sugar alcohols, diols, polyols, or polyether alcohols, fatty acids, organic solvents, waxes, oils, poloxamers, cyclodextrins, or any combination thereof. For example, the solubilizer may be glycerol, polyethylene glycol (such as PEG 3350), propylene glycol, poloxamer 124, poloxamer 407, Labrasol^{®} (caprylocaproyl polyoxyl-8 glycerides), Kleptose^{®} HPB, Captisol^{®} sulfobutylether β-cyclodextrin, or any combination thereof. In some embodiments, the water-miscible solubilizer is glycerol, propylene glycol, polyethylene glycol, or any combination thereof. For example, the water-miscible solubilizer may include both glycerol and propylene glycol.

The present formulations can also include a buffer that is effective to maintain the pH of the formulation at about 4.5 to about 9. For example, the buffer may be effective to maintain the pH at about 5.0 to about 9, about 5.0 to about 9, about 5.0 to about 8.5, about 5.0 to about 8.2, about 5.0 to about 8, about 5.0 to about 6.0, about 5.0 to about 5.5, about 5.2 to about 9, about 5.2 to about 8.5, about 5.2 to about 8.2, about 5.2 to about 8, about 5.2 to about 7, about 5.2 to about 6, about 5.2 to about 5.5, about 5.5 to about 9, about 5.5 to about 8.5, about 5.5 to about 8.2, or about 5.5 to about 8, or at about 4.5, about 4.6, about 4.7, about 4.8, about 4.9, about 5.0, about 5.1, about 5.2, about 5.3, about 5.4, about 5.5 about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7, about 7.8. about 7.9, about 8.0, about 8.1, about 8.2, about 8.3, about 8.4, about 8.5, about 8.6, about 8.7, about 8.8, about 8.9, or about 9.0. In certain embodiments, the pH of the formulation may be about 4.5 to about 7, for example about 4.5 to about 6, about 5 to about 6, or about 5.5, wherein "about" relates to +/-10% of the recited value.

In certain embodiments, the pH of the formulation may be about 7 or lower, such as about 6 or lower. Buffers that may be used to maintain the pH of the formulation at a desired level can be readily identified by those of ordinary skill in the art, and may include, for example, water, phosphate buffer, sodium phosphate, buffer, Tris buffer, or citrate buffer. In the present formulations, the buffer may be present in a quantity that, when combined with the other components of the formulation, brings the total amount of components to 100 wt%.

The present formulations are designed for topical application to a subject's skin. Accordingly, the formulations are not configured for oral administration or for injection. Put differently, the formulations are non-oral and non-injectable.

The formulations can include a volatile solvent that at least partially evaporates from the skin surface following application. For example, in certain embodiments, the buffer component is aqueous, and the water that is contained within the aqueous buffer represents the volatile solvent. The portion of the formulation that remains following at least partial evaporation can be referred to as the "nonvolatile" or "residual" phase, and the volatile element(s) of the formulation that evaporate from the skin surface represents the "volatile" phase. In the present formulations, the detomidine or salt thereof is at or close to its saturation point within the nonvolatile phase following evaporation of the volatile phase. For example, the detomidine or salt thereof may be present in the residual phase following topical application of the instant formulations at or greater than about 75% of the saturation point of the active agent. In some embodiments, the detomidine or salt thereof is present in the residual phase at or greater than about 77, about 80, about 82, about 84, about 85, about 87, about 88, about 90, about 92, about 94, about 95, about 96, about 97, about 98, or about 99% of the saturation point for the detomidine or salt thereof.

In certain embodiments, the formulations may include an inert excipient that assists with increasing the concentration of the detomidine or salt thereof in the residual phase following topical application. In effect, such excipients can cause "salting out" of the detomidine or salt thereof from the other components of the residual phase, which can have the effect of increasing the activity of the detomidine or salt thereof on the surface of the subject's skin and promote permeability of the drug through the skin. Such inert excipients can include, for example, a polyol or simple sugar, such as sucrose, dextrose, trehalose, mannitol, sorbitol, or xylitol.

As noted above, and as described more fully below, the present formulations provide prolonged, substantially non-systemic treatment for pain. The period of time over which the formulations can provide treatment for pain is up to 24 hours when topically applied once a day. In certain embodiments, the formulations are preferably applied twice per day, and in such instances the treatment of pain that is provided by a first of the two topical administrations has a duration that lasts until the second topical administration, and the second daily topical administration has a duration that lasts until the following day's first topical administration. As used herein, "substantially non-systemic" refers to a treatment effect that is localized to the bodily region (for example, body part) to which the formulation is topically applied, with a minimal or no medically relevant effect outside of that bodily region, or simply no minimal or no medically relevant systemic effect.

Being designed for topical application, the present formulations may take any appropriate form, including, for example, that of a cream, foam, gel, lotion, or ointment. As required and as described herein, the formulations according to the present disclosure may further comprise one or more additional components in order to produce the topical form, such as thickening or gelling agents, preservatives, antioxidants, permeation enhancers, emulsifying agents, emollients, or humectants.

Thickening or gelling agents can include, but are not limited to, hydroxymethyl cellulose, hydroxyethyl cellulose, hydroxypropyl cellulose, hydroxypropylmethyl cellulose, xanthan gum, carbomers (acrylates and acrylic acid and its derivatives polymers, such as Carbopol^{®} 980 (crosslinked polyacrylate polymer)), povidones (e.g., polyvinylpyrrolidone), Poloxamers, Polyamide-3 (e.g., OleoCraft^{™} HP33), and other appropriate agents.

Preservatives can include, but are not limited to, benzalkonium chloride, parabens, sorbic acid and its salts, benzoic acid and its salts, cetrimonium bromide and chloride salts, phenoxyethanol, and other agents.

Antioxidants can include, but are not limited to, sodium metabisulfite, ascorbic acid, tocopheryl acetate (for purely aqueous formulations), and BHT or BHA (for hydrophobic formulations).

Permeation enhancers can include, but are not limited to, Transcutol^{®} P (highly purified diethylene glycol monoethyl ether EP/NF) or dimethylisosorbide (DMI).

Emulsifying agents can include, but are not limited to, Tweens, Spans, poloxamers (124, 407, 188), Brij S2 and Brij 721, Crodex M (cetearyl alcohol and potassium cetyl Phosphate), Crodafos CES (cetearyl alcohol and dicetyl phosphate and Ceteth-10 phosphate (Crodafos CES), Cithrol DPHS (PEG 30 Dipolyhydroxystearate), cyclopentasiloxane, or macrogol hydroxystearate.

Emollients can include, but are not limited to, Migliol 810 or 812 (caprylic-capric triglycerides), Isoporpyl Isostearate (Crodamol IPIS), Isostearyl Isostearate (Crodamol ISIS), PPG-11 Stearyl Ether (Arlamol PS11E), Macrogol 6 Glycerol Caprylocaprate (Glycerox 767HC), or Labrasol^{®} (caprylocaproyl polyoxyl-8 glycerides).

Humectants can include, but are not limited to, glycerin, propylene glycol, 1,3-propanediol, or 1,2-pentanediol.

The present formulations may contain detomidine or a salt thereof as the only therapeutic agent. In other embodiments, the formulations may include a further therapeutic agent in addition to the detomidine or a salt thereof. For example, the formulations may further comprise an analgesic (such as an NSAID, an opioid, or acetaminophen), an antidepressant agent (such as a tricyclic antidepressant), an anticonvulsant agent, or a local anesthetic (such as lidocaine, prilocaine, tetracaine, benzocaine, proxymetacaine, and the like). As another example, the formulations may further comprise one or more additional α-2-adrenergic agonists. Preferred α-2-adrenergic agonists include clonidine, romifidine, brimonidine, dexmedetomidine, and salts thereof.

In certain embodiments, the formulations comprise up to about 1% of detomidine or salt thereof, and produce any one or more of: a blood plasma concentration of no more than about 500 pg/mL of the detomidine or salt thereof in the subject during the first 48 hours of twice-daily topical administration of said formulation; a blood plasma concentration of no more than about 500 pg/mL of the detomidine or salt thereof in the subject during the first 72 hours of twice-daily topical administration of the formulation; a blood plasma concentration of no more than about 500 pg/mL of the detomidine or salt thereof in the subject during the first 96 hours following topical application of the formulation on the subject; a blood plasma concentration of no more than about 800 pg/mL of the detomidine or salt thereof in the subject following the first 96 hours of twice-daily topical administration of the formulation; at least about 120 ng per mg of the subject's dermis per cm² of detomidine or salt thereof at any point during the first 24 hours of twice-daily topical administration of the formulation; at least about 180 ng per mg of the subject's dermis per cm² of detomidine or salt thereof at any point during the first 96 hours of twice-daily topical administration of the formulation; at least about 1200 ng per mg of the subject's epidermis per cm² of detomidine or salt thereof at any point during the first 24 hours of twice-daily topical administration of the formulation; at least about 4800 ng per mg of the subject's epidermis per cm² of detomidine or salt thereof at any point during the first 24 hours of twice-daily topical administration of the formulation; at least about 2000 ng per mg of the subject's epidermis per cm² of detomidine or salt thereof at any point during the first 96 hours and following the first 24 hours of twice-daily topical administration of the formulation; or, at least about 2400 ng per mg of the subject's epidermis per cm² of detomidine or salt thereof at any point during the first 96 hours and following the first 24 hours of twice-daily topical administration of the formulation.

Topical administration of the formulation once- or twice-daily to a subject for up to four days can result in a blood plasma concentration in the subject that is less than that required to achieve a systemic therapeutic effect of the detomidine or salt thereof.

In certain embodiments, the topical formulations comprise 0.01 to 5 wt% detomidine hydrochloride, glycerine, propylene glycol, a gelling agent, and a buffer that is effective to maintain the formulation at pH about 4.5 to about 8.2. In some embodiments, the topical formulations comprise 0.01 to 3 wt% detomidine hydrochloride, glycerine, propylene glycol, a cellulose gelling agent, and a buffer that is effective to maintain the formulation at pH about 4.5 to about 6. In some other embodiments, the topical formulations comprise 0.05 to 3 wt% detomidine hydrochloride, glycerine, propylene glycol, a cellulose gelling agent, and a buffer that is effective to maintain the formulation at pH about 5 to about 6. In yet other embodiments, the topical formulations comprise 0.1 to 2 wt% detomidine hydrochloride, glycerine, propylene glycol, a cellulose gelling agent, and a buffer that is effective to maintain the formulation at pH about 5 to about 5.5. In still other embodiments, the topical formulations comprise 0.1 to 1 wt% detomidine hydrochloride, glycerine, propylene glycol, hydroxyethyl cellulose, and a buffer that is effective to maintain the formulation at pH about 5 to about 5.5. In yet other embodiments, the topical formulations comprise 0.1 to 1 wt% detomidine hydrochloride, glycerine, propylene glycol, hydroxyethyl cellulose, and a buffer that is effective to maintain the formulation at pH about 5.2 to about 5.5. Any of these embodiments may further comprise a preservative. Furthermore, any of these embodiments may be effective to produce a depot of the detomidine or salt thereof in the subject's skin following topical application.

The present disclosure also pertains to topical formulations comprising about 0.001 to about 3 wt% detomidine or a salt thereof; and, a carrier suitable for topical application to the skin of a subject, wherein the formulation is effective to provide relief from pain in the subject while producing a maximum blood plasma concentration of no more than about 1600 pg/mL after four days of twice daily administration of said formulation.

In certain embodiments, the topical formulations comprise 0.01 to 1 wt% detomidine hydrochloride, a gelling agent, a water-miscible solubilizer, and a buffer that is effective to provide a pH of about 4.5 to about 7. In certain embodiments, the topical formulations comprise 0.01 to 1 wt% detomidine hydrochloride, a gelling agent, a water-miscible solubilizer, and a buffer that is effective to provide a pH of about 5 to about 6. In certain embodiments, the topical formulations comprise 0.01 to 0.5 wt% detomidine hydrochloride, a gelling agent, a water-miscible solubilizer, and a buffer that is effective to provide a pH of about 4.5 to about 7. In certain embodiments, the topical formulations comprise 0.01 to 0.5 wt% detomidine hydrochloride, a gelling agent, a water-miscible solubilizer, and a buffer that is effective to provide a pH of about 5 to about 6. In certain embodiments, the topical formulations comprise 0.01 to 0.5 wt% detomidine hydrochloride, a gelling agent, a water-miscible solubilizer, and a buffer that is effective to provide a pH of about 5.5. In certain embodiments, the topical formulations comprise 0.03 to 0.2 wt% detomidine hydrochloride, a gelling agent, a water-miscible solubilizer, and a buffer that is effective to provide a pH of about 4.5 to about 7. In certain embodiments, the topical formulations comprise 0.03 to 0.2 wt% detomidine hydrochloride, a gelling agent, a water-miscible solubilizer, and a buffer that is effective to provide a pH of about 5 to about 6. In certain embodiments, the topical formulations comprise 0.05 to 0.15 wt% detomidine hydrochloride, a gelling agent, a water-miscible solubilizer, and a buffer that is effective to provide a pH of about 4.5 to about 7. In certain embodiments, the topical formulations comprise 0.05 to 0.15 wt% detomidine hydrochloride, a gelling agent, a water-miscible solubilizer, and a buffer that is effective to provide a pH of about 5 to about 6. In certain embodiments, the topical formulations comprise about 0.1 wt% detomidine hydrochloride, a gelling agent, a water-miscible solubilizer, and a buffer that is effective to provide a pH of about 4.5 to about 7. In certain embodiments, the topical formulations comprise about 0.1 wt% detomidine hydrochloride, a gelling agent, a water-miscible solubilizer, and a buffer that is effective to provide a pH of about 5 to about 6. In certain embodiments, the topical formulations comprise about 0.1 wt% detomidine hydrochloride, a gelling agent, a water-miscible solubilizer, and a buffer that is effective to provide a pH of about 5.5. In certain embodiments, the topical formulations comprise about 0.1 wt% detomidine hydrochloride, about 1% to about 3% of a gelling agent, about 0.1% to about 50% of a water-miscible solubilizer, and a buffer that is effective to provide a pH of about 4.5 to about 7. In certain embodiments, the topical formulations comprise about 0.1 wt% detomidine hydrochloride, about 1% to about 3% of a gelling agent, about 0.1% to about 30% of a water-miscible solubilizer, and a buffer that is effective to provide a pH of about 4.5 to about 7. In certain embodiments, the topical formulations comprise about 0.1 wt% detomidine hydrochloride, about 1% to about 3% of a gelling agent, about 0.1% to about 30% of a water-miscible solubilizer, and a buffer that is effective to provide a pH of about 5 to about 6. In certain embodiments, the topical formulations comprise about 0.1 wt% detomidine hydrochloride, about 1% to about 3% of a gelling agent, about 0.1% to about 10% of a water-miscible solubilizer, and a buffer that is effective to provide a pH of about 4.5 to about 7. In certain embodiments, the topical formulations comprise about 0.1 wt% detomidine hydrochloride, about 1% to about 3% of a gelling agent, about 0.1% to about 1% of a water-miscible solubilizer, and a buffer that is effective to provide a pH of about 4.5 to about 7. In certain embodiments, the topical formulations comprise about 0.1 wt% detomidine hydrochloride, about 1 % to about 3% of a gelling agent, about 0.1% to about 1 % of a water-miscible solubilizer, and a buffer that is effective to provide a pH of about 5 to about 7. In certain embodiments, the topical formulations comprise about 0.1 wt% detomidine hydrochloride, about 1% to about 3% of a gelling agent, about 0.1% to about 1% of a water-miscible solubilizer, and a buffer that is effective to provide a pH of about 5 to about 6. In certain embodiments, the topical formulations comprise about 0.1 wt% detomidine hydrochloride, about 1% to about 3% of a gelling agent, about 0.1% to about 1% of a water-miscible solubilizer, and a buffer that is effective to provide a pH of about 5.5. In certain embodiments, the topical formulations comprise about 0.1 wt% detomidine hydrochloride, about 1% to about 3% of hydroxyethyl cellulose, about 0.1% to about 1% of glycerin, and a buffer that is effective to provide a pH of about 5 to about 6. In certain embodiments, the topical formulations comprise about 0.1 wt% detomidine hydrochloride, about 1% to about 3% of hydroxyethyl cellulose, about 0.1% to about 1% of glycerin, and a buffer that is effective to provide a pH of about 5.5. In certain embodiments, the topical formulations comprise about 0.1 wt% detomidine hydrochloride, about 1.75% of hydroxyethyl cellulose, about 0.3% of glycerin, and a buffer that is effective to provide a pH of about 5 to about 6. In certain embodiments, the topical formulations comprise about 0.1 wt% detomidine hydrochloride, about 1.75% of hydroxyethyl cellulose, about 0.3% of glycerin, and a buffer that is effective to provide a pH of about 5.5.

As well as a water-miscible solubilizer, the carrier component of the present formulations can further comprise a buffer. Unless otherwise specified below, such formulations may share any one or more of the characteristics described above with respect to the other inventive formulations, including the quantity of the detomidine or salt thereof; the identity of possible salt forms of the detomidine; the presence, and, the amount and type of possible water-miscible solubilizers; the pH of the formulation; the identity of possible buffers for maintaining the desired pH; the percentage of detomidine or salt thereof relative to its saturation point in the residual phase of the formulation following application to the skin; the presence and identity of optional additional therapeutic agents; the presence of an inert excipient that assists with increasing the concentration of the detomidine or salt thereof in the residual phase following topical application; and, the presence and identity of optional additional components other than a further therapeutic agent or inert excipient. Thus, the detailed description of these characteristics provided above can apply to the present formulations, as well.

In certain embodiments, the formulations comprise up to 1% of detomidine or salt thereof, and produce any one or more of: a maximum blood plasma concentration of no more than about 1400 pg/mL after three days of twice daily administration of the formulation; a maximum blood plasma concentration of no more than about 1200 pg/mL after two days of twice daily administration of the formulation; a maximum blood plasma concentration of no more than about 800 pg/mL after the first day of twice daily administration of the formulation; at least about 120 ng per mg of the subject's dermis per cm² of detomidine or salt thereof at any point during the first 24 hours of twice daily topical administration of the formulation; at least about 180 ng per mg of the subject's dermis per cm² of detomidine or salt thereof at any point during the first 96 hours of twice-daily topical administration of the formulation; at least about 1200 ng per mg of the subject's epidermis per cm² of detomidine or salt thereof at any point during the first 24 hours of twice-daily topical administration of the formulation; at least about 4800 ng per mg of the subject's epidermis per cm² of detomidine or salt thereof at any point during the first 24 hours of twice-daily topical administration of the formulation; at least about 2000 ng per mg of the subject's epidermis per cm² of detomidine or salt thereof at any point during the first 96 hours and following the first 24 hours of twice-daily topical administration of the formulation; or, at least about 2400 ng per mg of the subject's epidermis per cm² of detomidine or salt thereof at any point during the first 96 hours and following the first 24 hours of twice-daily topical administration of the formulation.

Topical administration of the formulations once- or twice-daily to a subject for up to four days can result in a blood plasma concentration in the subject that is less than that required to achieve a systemic therapeutic effect of the detomidine or salt thereof. According to one embodiment, topical administration of the formulations once- or twice-daily to a subject for at least four days results in a blood plasma concentration in the subject that remains less than that required to achieve a systemic therapeutic effect of the detomidine or salt thereof. Preferably, the topical administration can continue for weeks, months, or longer while maintaining a subtherapeutic systemic blood plasma concentration.

The present invention also provides compositions for use in treating pain in a subject in need thereof comprising topically administering to the subject an effective amount of a formulation according to any one of the embodiments disclosed herein. The present disclosure also provides compositions for use in providing prolonged, non-systemic treatment for pain in a subject in need thereof comprising topically administering to the subject a formulation according to any one of the embodiments disclosed above. In certain embodiments, the pain treated according to the methods of the present invention is neuropathic pain. In certain embodiments, the neuropathic pain is postherpetic neuralgia. In certain embodiments, the neuropathic pain is diabetic peripheral neuropathy.

Also provided herein are methods for forming a subcutaneous (*i.e.,* below skin surface) depot of detomidine or a salt thereof in a subject comprising topically administering to the subject a formulation according to any one of the above-described embodiments, wherein the detomidine or salt thereof is released from the depot into the subject in order to provide prolonged, non-systemic treatment of pain in the subject. Pursuant to such methods, the subcutaneous depot of detomidine or salt thereof may form in the subcutaneous tissue (hypodermis), the epidermis, or the dermis of a subject's skin. The depot can release a sufficient quantity of the detomidine or salt thereof to the patient such that administration of the formulation to the subject on a once- or twice-daily basis is sufficient to provide the prolonged, non-systemic treatment of pain in the subject. In certain embodiments, the depot forms in a least the epidermal and dermal layers of the subject's skin. In further embodiments, a higher concentration of detomidine is observed in the epidermal layer, as compared to the dermal layer.

The pain for which any of the above methods provide treatment can be any type of pain for which topical treatment is relevant, whether acute or chronic. For example, the pain for which treatment is provided using the present methods may be somatic (caused by the activation of pain receptors in either the body surface or musculoskeletal tissues, such as postsurgical pain), visceral (caused by damage or injury to internal body structures or organs), or neuropathic (postherceptic neuralgia and diabetic neuropathy representing examples). Exemplary types of pain that can be treated according to the present methods include carpal tunnel syndrome, abdominal pain, hip pain, knee and other joint pain, pain deriving from piriformis syndrome, back pain, neck or shoulder pain, acute or chronic muscle pain, trigeminal neuralgia, postherceptic neuralgia, sciatica pain, arachnoiditis (spinal pain), pain from complex regional pain syndrome, phantom limb pain diabetes-related nerve pain (neuropathy), pain deriving from depression or anxiety, and pain from compartment syndrome.

In accordance with the presently disclosed methods, the topical administration may be performed using conventional techniques. For example, the administration may be performed by delivering an aliquot of the formulation to a physician's or subject's hand (preferably gloved), which is used to smear and then rub the formulation onto an area of skin on the body part for which treatment is desired. The formulation may be topically administered in the chosen manner on a once-daily or twice-daily basis. When the method comprises applying the formulation on a twice-daily basis, appropriate temporal spacing between applications should be used. For example, if the subject is awake for a 16 hour period of the day, then a first application can be performed in the morning, and a second application can be performed in the evening, for example, prior to retiring to bed.

The present invention also includes modified versions of each topical formulation described herein, which are defined by the claims and to compositions for use in topically administering and treating pain described herein using such

### EXAMPLES

The following examples are set forth so as to provide those of ordinary skill in the art with a complete disclosure and description of how the methods, compositions, and components claimed herein are made and evaluated, and are intended to be purely exemplary of the invention and are not intended to limit the scope of what the inventors regard as their invention. Examples 1-15B and 19-21 all relate to comparative examples.

### Example 1 - Pharmacokinetic Analysis of Single Dose of 0.1% Detomidine Formulations

Topical formulations containing detomidine HCl were prepared in order to test in vivo permeability and pharmacokinetics using a minipig *in vivo* model. The tested formulations are described in Table 1, below.

**Table 1**

| | Gel | | | | | Ointment |
|---|---|---|---|---|---|---|
| Formulation | pH 6.2 | pH 7.2 | pH 8.2 | pH 7.2 | pH 7.2 | Dispersed DS Ointment |
| Batch No. | R-12074 | R-12083 | R-12081 | R-12148 | R-12522 | R-12150 |
| Detomidine HCl | 0.1% | 0.1% | 0.1% | 0.1% | 1.0% | 0.1% |
| Hydroxyethyl cellulose (Natrosol 250HH) | 1.75% | 1.75% | 1.75% | 1.75% | 1.75% | --- |
| Propylene Glycol | --- | 30.0% | --- | 30.0% | 30.0% | --- |
| Glycerin | 30.4% | 0.4% | 30.4% | 0.4% | 4.0% | --- |
| Transcutol P | --- | --- | --- | 10.0% | 10.0% | --- |
| Methyl paraben | 0.15% | 0.15% | 0.25% | 0.15% | 0.15% | --- |
| Propyl paraben | 0.15% | 0.15% | 0.05% | 0.15% | 0.15% | --- |
| Phosphate Buffer (0.1M) | Ad 100% | Ad 100% | --- | Ad 100% | Ad 100% | --- |
| Tris Buffer (0.1M) | --- | --- | Ad 100% | --- | --- | --- |
| Migliol 810 | --- | --- | --- | --- | --- | 20.0% |
| White Petrolatum | --- | --- | --- | --- | --- | Ad 100% |

The study design was as follows: Gottingen minipigs, n=3, single dose applied for 24 hrs. Dose: 5 µl/cm², 0.22 mg/kg/day, dosing 10% of the BSA equals 302.44 cm² in 7 kg minipig. About 1.5 mg of detomidine HCl applied to each minipig. Blood samples were taken at: 1, 2, 4, 6, 8, 12, 18, 24, 30, 36, 48, 72, 96, 120, 144 and 168 hours post-treatment. Bioanalysis: Detomidine (LLOQ-25 pg/ml) and major metabolite Carboxy-detomidine (LLOQ 50 pg/ml). Skin biopsies for PK analysis were taken at 12 and 24 hrs. Stratum corneum, epidermis and dermis layers were analyzed separately. Skin biopsies for histopathological evaluation were taken at 24 and 168 hrs.

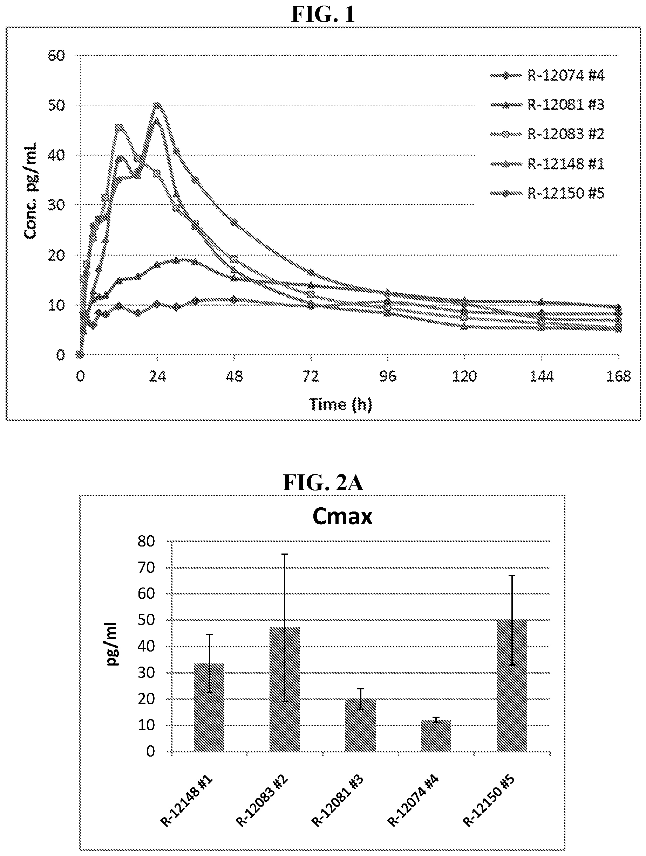

The blood plasma concentration curves for all tested formulations (including those yielding values that were below the quantifiable limit (BLQ)) are shown in FIG. 1. The lowest plasma levels and the slowest penetration rate resulted from the glycerine-based gels (batches R-12074 and R-12081). The propylene glycol (PG)-based formulations penetrated faster, achieving Tmax by 12-24 hrs. The penetration of the ointment was comparable to the PG-based gels.

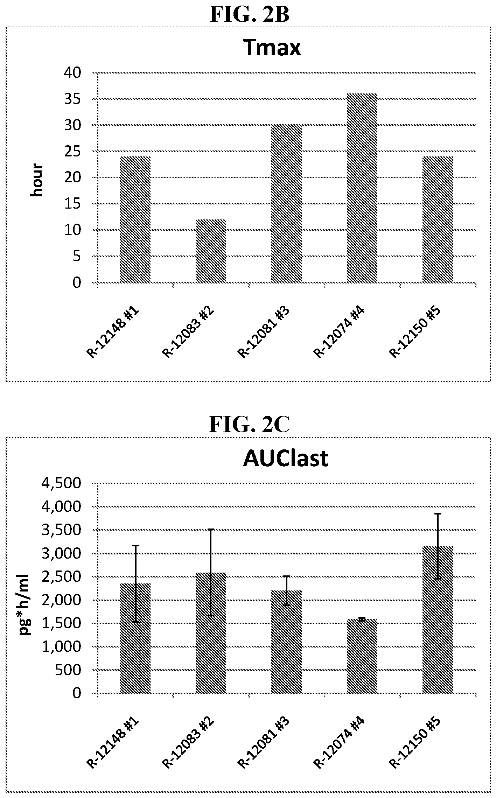

FIG. 2A, 2B, and 2C provide the results of an analysis of pharmacokinetic parameters (Cmax, Tmax, and AUClast, respectively) resulting from testing of inventive formulations for the ability to avoid systemic effect. The formulations R-12074 and R-12081, formulated with glycerin, showed lower systemic exposure as compared to the ointment (R-12150) or the formulations containing propylene glycol (PG). The lowest systemic exposure was achieved with R-12074 (containing glycerine). A comparable systemic exposure was achieved with or without Transcutol (penetration enhancer). The penetration properties of the ointment were comparable to the formulation based on PG + transcutol.

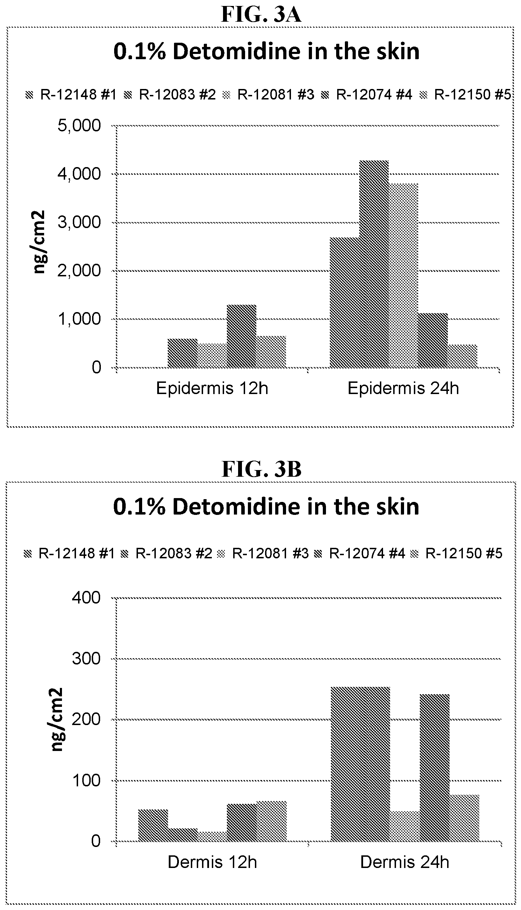

FIG. 3A and FIG. 3B show the results of an evaluation of the degree of accumulation of detomidine HCl in epidermis and dermis, respectively, following application of the inventive formulations described in Table 1, as expressed in terms of ng/cm². By 12 h, all of the formulations showed a comparable penetration and accumulation. At 24 h, an increase in the drug accumulation was achieved as compared to the 12 h (excluding the ointment). A high CV% was obtained for all formulations. Table 2, below, provides the coefficient of variation percentage for each of the tested formulations in the epidermis and dermis at 12 and 24 hours.

**Table 2**

| | Epidermis 12h | Dermis 12h | Epidermis 24h | Dermis 24h |
|---|---|---|---|---|
| | VC% | | | |
| R-12148 #1 | 121 | 167 | 36 | 110 |
| R-12083 #2 | 121 | 76 | 91 | 96 |
| R-12081 #3 | 89 | 99 | 54 | 11 |
| R-12074 #4 | 161 | 113 | 85 | 127 |
| R-12150 #5 | 85 | 75 | 173 | 113 |

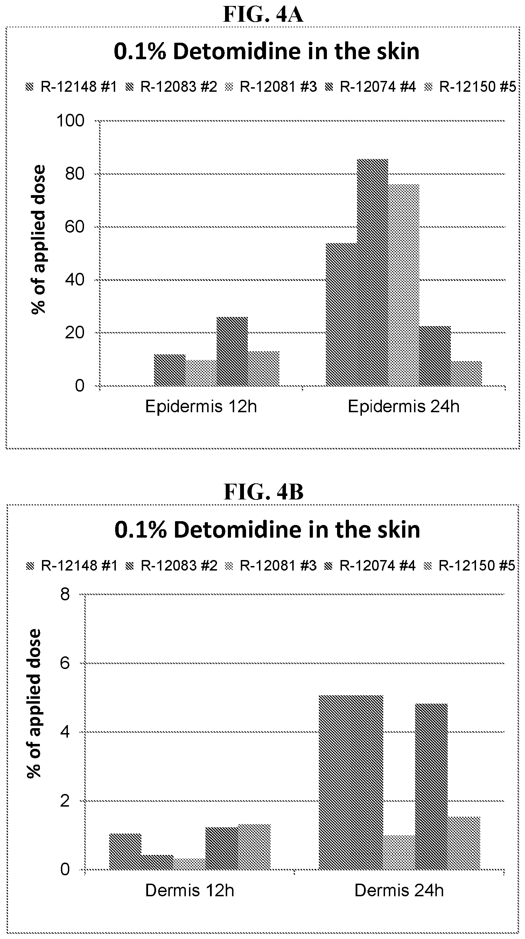

FIGS. 4A and FIG. 4B show the results of an evaluation of the degree of accumulation of detomidine HCl in epidermis and dermis, respectively, as expressed in terms of percentage of applied dose. After 12 h, 10-26% of the applied dose penetrated into epidermis, and 0.3-1.3% of the applied dose penetrated into dermis. At 24 h, between 9-85% of the applied dose penetrated into epidermis, and 1-5% of the applied dose penetrated into dermis. This data showed that a high proportion of the applied dose could be found in the skin 24 hours later.

### Example 2 - In Vivo Testing in Post- Operative Pain Model in Pigs

An evaluation of inventive topical formulations containing detomidine using von Frey testing, which measures the force applied to the painful area of interest that induces the subject to withdraw from the stimulus (pain response), was conducted. The greater the measured force, the higher the efficacy of the analgesic formulation. There were six pigs per test group. A 6-7 cm long skin and fascia incision was made in the left flank of each test animal, keeping the muscle intact. The incision was at the area in which the fascia is homogeneous. The skin incision was then closed using a sterile suture. Each formulation was applied at a dose of about 3 µL/cm² close to the incision (total applied = about 150 µL). The formulations were not applied directly on the incision. The total test period was five days, with each formulation being applied on the day of surgery (day 0) and then twice per day for the following four days (days 1-4), once in the morning and again about 6-8 hours later. Naropin^{®} (Ropivacaine 1%) was used as a positive control in a separate group of 6 animals, and was administered once daily (5 mL SC = 50 mg/dose).

Post operative pain was assessed using the Von Frey methodology. Von Frey filaments (Ugo Basile) were applied at approximately ~0.5 cm proximal to the incision line to the surface of the flank skin. As the gram number of filaments increases, the force on the flanks' skin increases. The maximum force is 60 g. Filaments were applied until the animal withdrew from the stimuli. Each filament was applied 3-5 times. If withdrawal was not achieved, a thicker filament was applied. If a withdrawal was achieved, a thinner filament was applied (thicker or thinner refers to higher/thicker or lower/thinner gram force). By alternating (3 times) the filament thickness, the force required to achieve withdrawal reaction was determined and recorded. Withdrawal reaction was considered as the act of moving away from the stimuli - either by walking away or by twisting of the flank. Tail waving alone was not considered as a withdrawal reaction. The stimulus was applied on the flank while the pigs were being fed by a specifically assigned researcher to whom the animals were acclimatized. This procedure was carried out on study day 0, at 1,3,5 and 7 hours post surgery. Then again 1 h pre- and post-AM dosing on study days 1-4. The before-dosing testing was intended to assess the duration of effect between doses and the after dosing testing assessed the effect immediately after dosing and any accumulation multiple dosing effect. Blood was drawn at each time point, and at the end of the study, blood and tissue were collected. The tested formulations are shown in Table 3, below:

**Table 3**

| Batch No. | R-12693 | R-12635 | R-12668 | R-12669 | R-12694 | R-12418 |
|---|---|---|---|---|---|---|
| Detomidine HCl | 0.33% | 1.0% | 0.33% | 1.0% | - | - |
| Clonidine HCl | - | - | - | - | 1.0% | - |
| Hydroxyethyl cellulose (Natrosol 250HH) | 1.75% | 1.75% | 1.75% | 1.60% | 1.75% | 1.75% |
| Propylene Glycol | - | - | 30.0% | 40.0% | 30.0% | - |
| Glycerin | 30.0% | 30.0% | - | - | - | 30.4% |
| Methyl paraben | 0.25% | 0.25% | 0.15% | 0.15% | 0.15% | 0.15% |
| Propyl paraben | - | - | 0.15% | 0.15% | 0.15% | 0.15% |
| Sodium Phosphate Buffer (0.1M) | pH 6.2 Ad 100% | pH 6.2 Ad 100% | pH 7.2 Ad 100% | pH 7.2 Ad 100% | - | pH 6.2 Ad 100% |
| Tris Buffer (0.1M) | - | - | - | - | pH 8.2 Ad 100% | - |

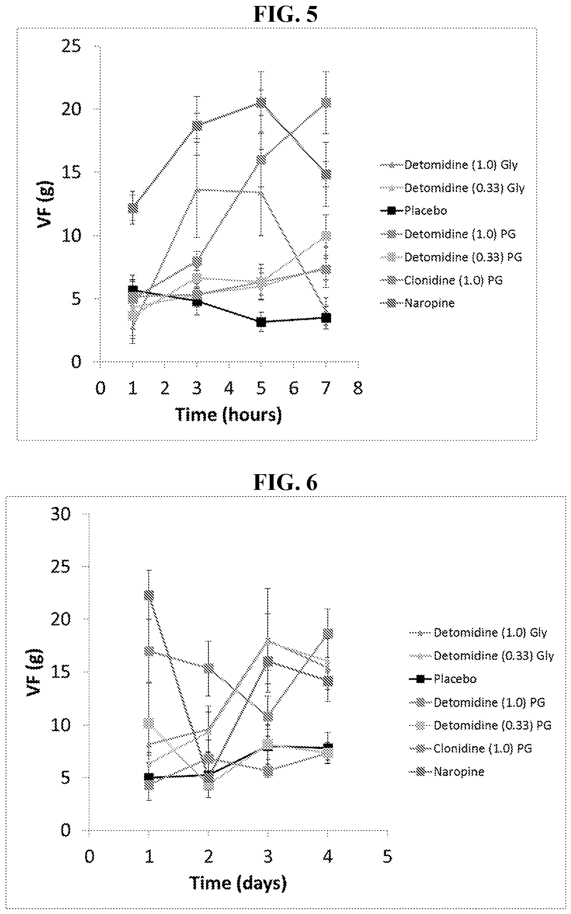

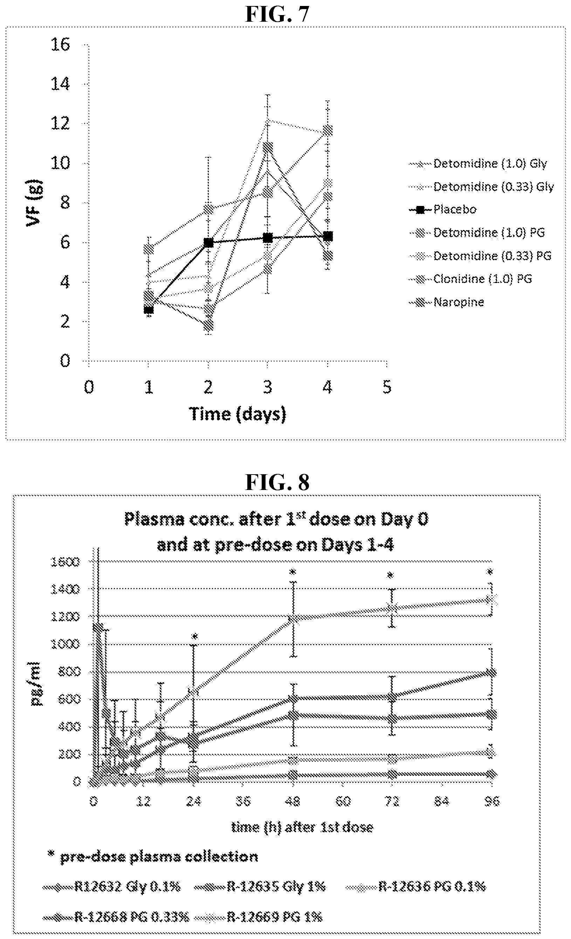

FIG. 5 shows the von Frey response with respect to each of the tested formulations as measured during the first few hours following topical application immediately after surgery. The scale is in hours following application of the formulations. FIG. 6 shows the von Frey response with respect to each of the tested formulations as measured one hour following each topical application over four days. FIG. 7 shows the von Frey response with respect to each of the tested formulations as measured 15 hours following the preceding evening's dose, and before the first dose of the current day, over four days.

This pharmacodynamics study was conducted in domestic swine using the Post-Operative Pain model. Two different inventive formulations of detomidine, each at two different dose strengths (0.33% and 1.0%), were used and administered topically, b.i.d., in a blinded manner. They were compared to clonidine (1%) in PG and to Naropin (ropivacaine). Pain was measured using von Frey filaments. Before surgery, the minimum force measured by von Frey to evoke a response was 60 grams. After surgery, the average force necessary was around 4 grams, staying constant during the 5 day study.

The positive control, Ropivacaine, consistently reduced pain sensitivity as evidenced by 12-23 grams being required to induce a pain response (avoidance) over the course of five days. Clonidine, over the course of the week, also reached 20g of force necessary to evoke a response.

Detomidine, in glycerin formulation, effectively attenuated pain in pigs compared to placebo as measured by von Frey testing. 1% detomidine in glycerin and 0.33% detomidine in glycerin were virtually identical in their efficacy over the 5 day period, requiring 18g force to cause the pigs to feel the von Frey filaments. Two-way RM ANOVA showed no significant difference due to treatment between these two groups as well as between them and the ropivacaine and clonidine formulations.

During the first day after surgery, 1% detomidine in glycerin was effective within 3 hours of administration. The glycerin formulation showed a dose-response effect that was not seen with the PG formulation, which had less efficacy.

### Example 3 - Pharmacokinetic Analysis of Additional Detomidine Formulations

Additional topical formulations containing detomidine HCl were prepared in order to test pharmacokinetics using a minipig *in vivo* model. The tested formulations and study design are described in Tables 4 and 5, below.

**Table 4**

| Formulation | R-12632 | R-12635 | R-12636 | R-12668 | R-12669 |
|---|---|---|---|---|---|
| Detomidine HCl | 0.10% | 1.00% | 0.10% | 0.33% | 1.00% |
| Hydroxyethyl cellulose (Natrosol 250HH) | 1.75% | 1.75% | 1.75% | 1.75% | 1.60% |
| Propylene Glycol | - | - | 30.00% | 30.00% | 40.00% |
| Glycerin | 30.00% | 30.00% | - | - | - |
| Methyl paraben | 0.25% | 0.25% | 0.15% | 0.15% | 0.15% |
| Propyl paraben | 0.05% | - | 0.15% | 0.15% | 0.15% |
| Phosphate Buffer (0.1M) | pH 6.2 Ad 100% | pH 6.2 Ad 100% | pH 7.2 Ad 100% | pH 7.2 Ad 100% | pH 7.2 Ad 100% |

**Table 5**

| **Group** | **Formulation** | **Animals per group** | **Detomidine concentration (dose)** | **Dose volume (mg/cm²)** | **BSA(%)** |
|---|---|---|---|---|---|
| 1 | R-12632 | 3 | 0.1% (0.44 mg/kg/day) | 5 | 10 |
| 2 | R-12635 | 3 | 1% (4.4 mg/kg/day) | 5 | 10 |
| 3 | R-12636 | 3 | 0.1% (0.44 mg/kg/day) | 5 | 10 |
| 4 | R-12668 | 3 | 0.33% (1.45 mg/kg/day) | 5 | 10 |
| 5 | R-12669 | 4 | 1% (4.4 mg/kg/day) | 5 | 10 |

Study design. Five formulations (Gly with 0.1% and 1% detomidine, and PG with 0.1%, 0.33%, and 1% detomidine) were repeatedly applied twice daily to groups female minipigs. Plasma was collected during the treatment period and up to 14 days (336 hours) after the last dose application. After 24h, only the pre-dose samples were collected. Skin biopsies were collected at pre-dose, 24h, 48h, 72h after the first dose, and at 12h and 14 days after the last dose application. ECG was recorded at pre-dose and 24h after the first dose and at 12h after the last dose application. Safety assessments also included clinical signs observation, evaluation of skin reactions at the application site and histopathological examination of skin biopsies collected 24h after the first dose and 60h after the last dose. Stratum corneum, epidermis and dermis layers were analyzed separately.

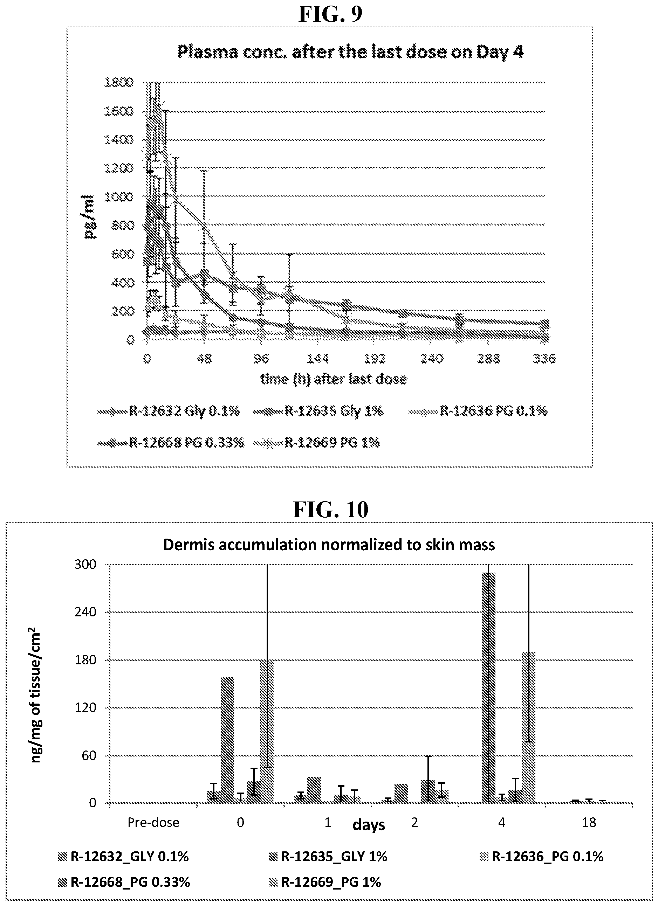

FIG. 8 shows the plasma concentration that was measured with respect to each formulation over time following a first dose on Day 0 and prior to the first dose on each of Days 1-4. FIG. 9 shows plasma concentrations that were measured with respect to the test formulations over time following a final dose on the fourth day of the pharmacokinetic evaluation. These results show that the plasma levels were dose-proportional for both glycerin and propylene-based formulations. PG-based formulations showed a higher extent of penetration as compared to Gly-based formulations achieving 2- and 1.4-fold higher Cmax and AUC, respectively. The highest plasma levels of up to 1.6 ng/ml were produced by the PG 1% formulation. A comparable Cmax was achieved with Gly 1% and PG 0.33% formulations, but the AUC was 1.7-fold higher for Gly 1%. The glycerine-based formulations showed a slower apparent rate of elimination compared with the PG-based formulations. Without being bound to any particular theory of operation, it is possible that the glycerine-based formulations resulted in the formation of a more substantial depot from which the detomidine was gradually released, thereby producing a slower apparent elimination rate.

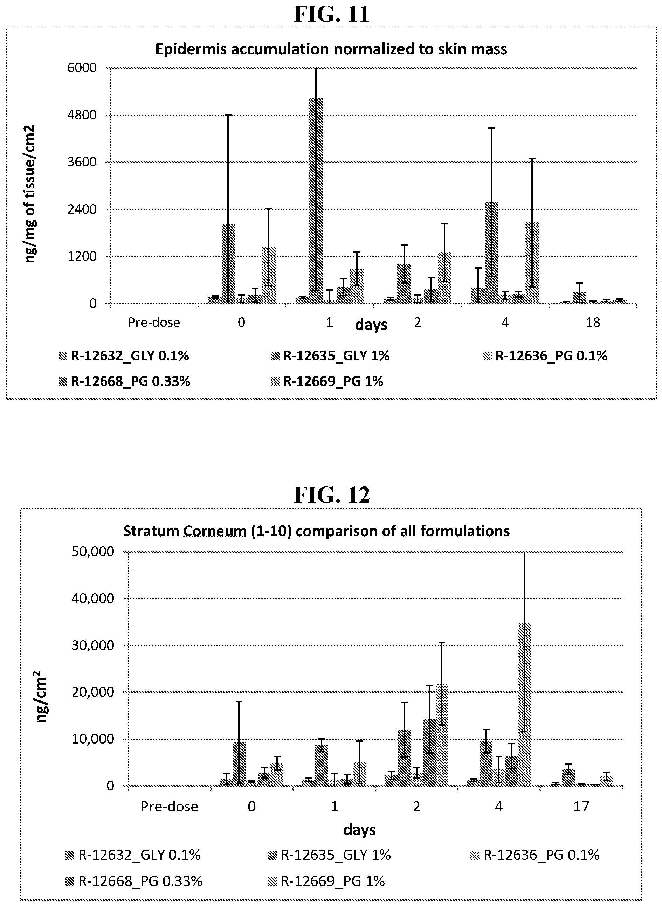

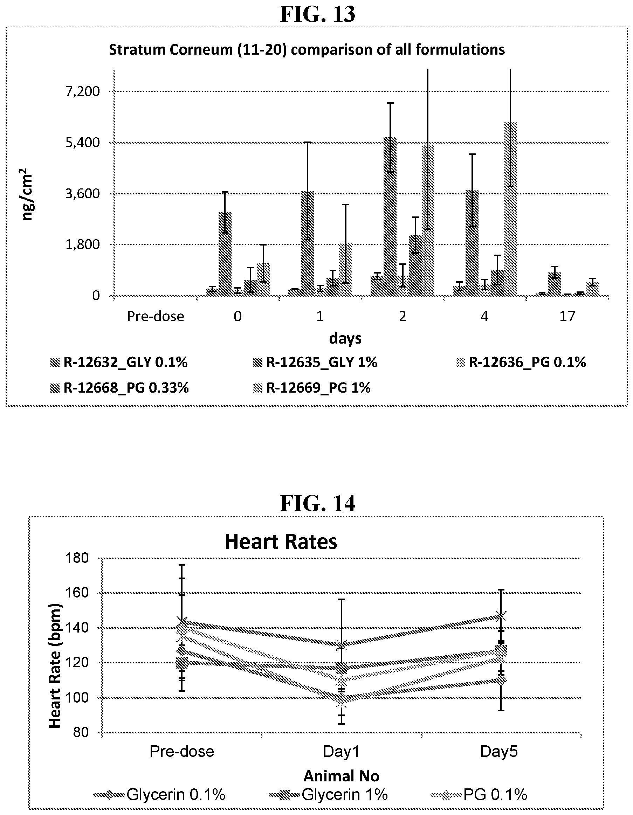

FIG. 10 shows the results of an assessment of the degree of accumulation of detomidine over time in the dermis resulting from application of the test formulations. FIG. 11 shows the results of an assessment of the degree of accumulation of detomidine over time in the epidermis. The drug levels in the epidermis were 10-100 fold higher than in the dermis. Minimal difference was observed between the Gly and PG-based formulations, but it appears that higher detomidine levels were achieved with 1% strength both in dermis and epidermis; However, the comparison of the drug levels in dermis and epidermis from different formulations should be treated with circumspection due to high variability across the animals. The dosedependent penetration through the skin can be supported by the higher levels achieved with 1% formulations (PG and Gly) in the deeper layers of stratum corneum. FIG. 12 shows the results of an assessment of the degree of accumulation of detomidine over time in the stratum corneum, strips 1-10, resulting from application of the test formulations, and FIG. 13 shows the results of an assessment of the degree of accumulation of detomidine over time in the stratum corneum, strips 11-20, resulting from application of the test formulations. For purposes of these investigations, higher strip numbers correspond to deeper (lower) levels of stratum corneum. These results show that detomidine levels in stratum corneum strips 1-10 were 10-fold higher than in strips 11-20, and compared to epidermis. Higher concentrations of drug were achieved with Gly 1% in strips 11-20.

FIG. 14 shows the results of an assessment of heart rate of the test pigs over time following administration of the test formulations. The data show that, from a safety perspective, no negative cardiovascular effects were recorded, *i.e*., no drops in heart rates, and no visible skin irritation was observed due to any of the treatments.

### Example 4 - In Vivo Testing of Additional Formulations in Post-Operative Pain Model in Pigs

An evaluation of additional topical formulations containing detomidine using von Frey testing (similar to described above in Example 2, but including day 5), was conducted. The dosing was twice daily. The von Frey measurements were conducted at 1, 3, 5 and 7 hours after application on the first day. On the consecutive days, the von Frey measurements were conducted 1 hour after the morning dosing but not 1 hour before. The tested formulations and placebo are described in Table 6, below:

**Table 6**

| Detomidine Concentration | 1.00% | 0.10% | Placebo | 0.033% | 0.01% |
|---|---|---|---|---|---|
| Batch number | R-14432 | R-14516 | R-14513 | R-14519 | R-14520 |
| Detomidine HCl | 1.00% | 0.10% | - | 0.03% | 0.01% |
| Hydroxyethyl cellulose (Natrosol 250HH) | 1.75% | 1.75% | 1.75% | 1.75% | 1.75% |
| Glycerin | 3% | 0.30% | 3.00% | 0.30% | 0.30% |
| Propylene Glycol | 1% | 0.10% | 1.00% | 0.10% | 0.10% |
| Transcutol P | - | - | - | 0.10% | 0.10% |
| Benzalkonium Chloride as 0.5% sol in Water | 0.02% | 0.02% | 0.02% | 0.02% | 0.02% |
| Buffer Citrate 0.2M | - | - | 25% | - | - |
| Ad 100% | buffer citrate 0.2M pH 5.2 | buffer citrate 0.05M pH 5.5 | water | buffer phosphate 0.05M pH 7.2 | tris buffer 0.05M pH 8.2 |

A positive control formulation containing 1.0% Naropin^{®} (ropivacaine HCl) 5 mL SC injection and a comparator formulation containing 0.10% clonidine (the latter otherwise having the characteristics of Batch Number R-14515), were also prepared. A formulation (batch R-14515) was also prepared comprising 0.33% detomidine HCl, 1.75% hydroxyethyl cellulose (Natrosol 250HH), 1% glycerin, 0.33% propylene glycol, 0.02% benzalkonium chloride as 0.5% sol in water, and citrate buffer 0.05 M pH 5.5 to 100%, but this formulation was not tested.

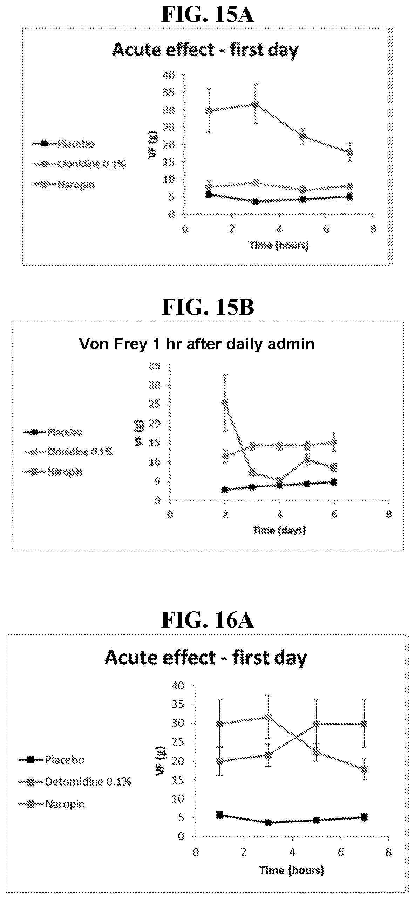

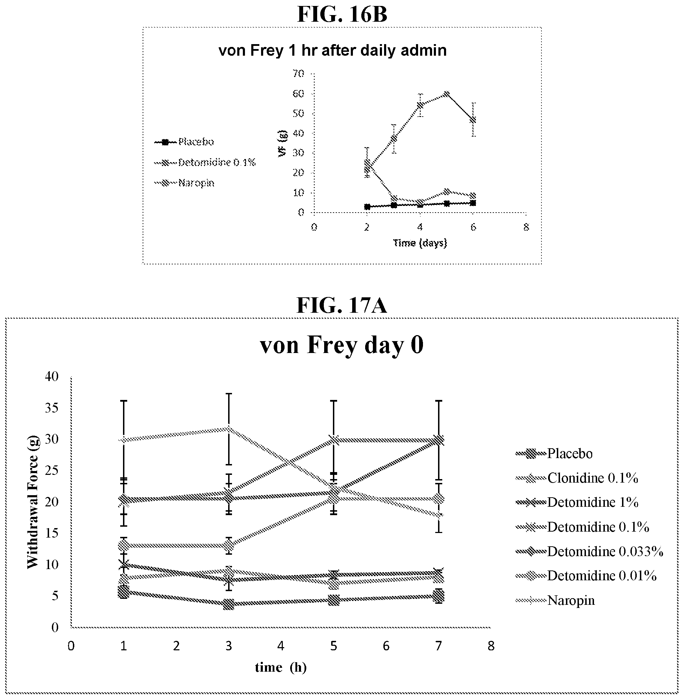

FIG. 15A shows the von Frey response with respect to the formulation containing 0.1% clonidine, to Naropin^{®} (ropivacaine HCl), and to the placebo formulation as measured following topical application immediately after surgery. The scale is in hours following application of the formulations. FIG. 15B shows the von Frey response with respect to each of these materials as measured one hour following each topical application over five days. FIG. 16A provides a comparison among the von Frey responses with respect to the formulation containing 0.1% detomidine, the Naropin^{®} (ropivacaine HCl), and the placebo formulation as measured following topical application immediately after surgery. The scale is in hours following application of the formulations. FIG. 16B shows the von Frey response with respect to each of these formulations as measured one hour following each topical application over five days.

These results demonstrate that the 0.1% detomidine formulation was at least as effective as the positive control on the first day, and provided considerably better results over an extended period of time.

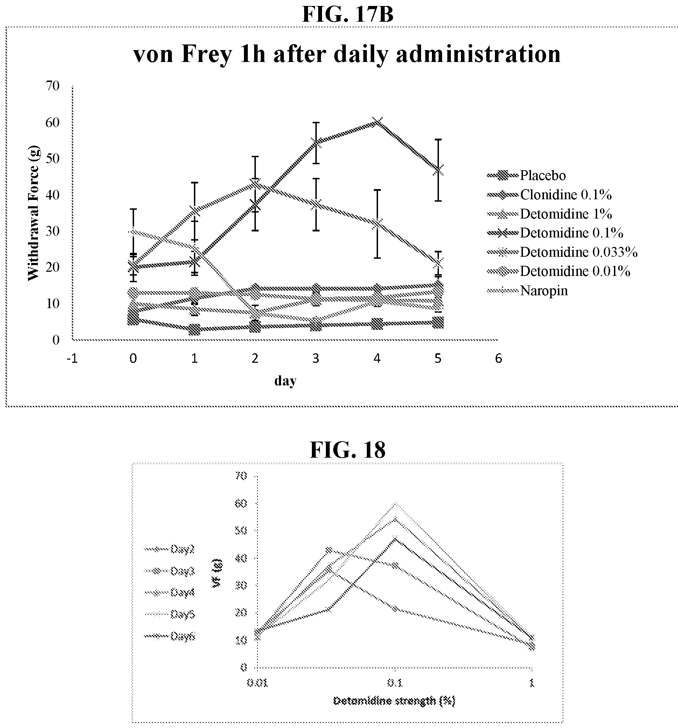

FIG. 17A provides a comparison among the von Frey responses with respect to all experimental formulations, as well as the placebo formulation, the Naropin^{®} (ropivacaine HCl), and the 0.1% clonidine formulation, as measured following topical application immediately after surgery. The scale is in hours following application of the formulations. FIG. 17B shows the von Frey response with respect to each of these formulations as measured one hour after each topical application over five days. FIG. 18 provides a comparison of the von Frey responses among the dose strengths over all days of the study. These comparisons demonstrate the bell shape efficacy response of the different detomidine strength formulations.

### Example 5 - In Vivo Testing of Additional Formulations in Post- Operative Pain Model in Pigs

An evaluation of additional topical formulations containing detomidine using von Frey testing (similar to described above in Example 4), was conducted. The tested formulations and placebo are described in Table 7, below:

**Table 7**

| | R-13392 | R-13391 | R-13558 | R-13510 | R-13557 | R-13556 | R-13559 |
|---|---|---|---|---|---|---|---|
| Detomidine HCl | 0.10% | 0.33% | 1.00% | - | - | - | - |
| Clonidine HCl | - | - | - | - | 0.10% | 1.00% | 0.10% |
| Hydroxyethyl cellulose (Natrosol 250HH) | 1.75% | 1.50% | 1.50% | 1.50% | 1.75% | 1.50% | - |
| Carbopol 980 | - | - | - | - | - | - | 1.00% |
| Glycerin | 12% | 20% | 20% | 20% | 3% | 10% | 2% |
| Propylene Glycol | 3% | - | - | - | 3% | 10% | 1% |
| PEG 3350 | 7% | 12% | 12% | 12% | 7% | 12% | - |
| Benzalkonium Chloride | 0.20% | 0.20% | 0.02% | 0.02% | 0.02% | 0.02% | - |
| Methyl Paraben | - | - | - | - | - | - | 0.10% |
| NaOH | - | - | 0.13% | - | 0.01% | 0.14% | About 2.75% to pH 8.0 |
| Phosphate buffer 0.1M pH 6.4 | - | - | 10% | - | - | - | - |
| Buffer Ad 100% | Tris 0.4M pH 8.2 | Tris 0.4M pH 8.2 | Water | Phosphate 0.1M pH 6.4 | Carbonate 0.1M pH 9.2 | Carbonate 0.1M pH 9.2 | Water |

A positive control formulation containing 1.0% Naropin^{®} (ropivacaine HCl) 5 mL SC injection was also prepared.

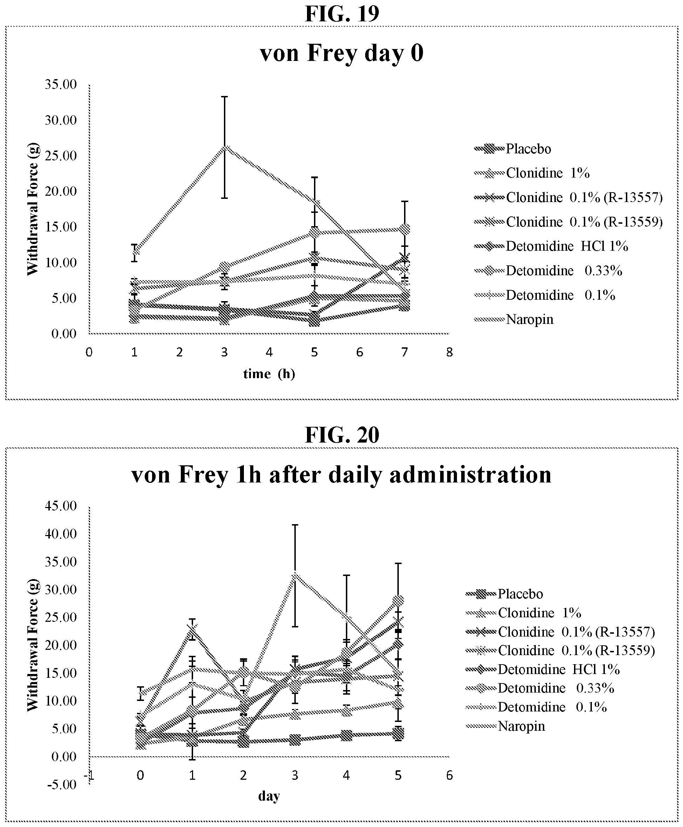

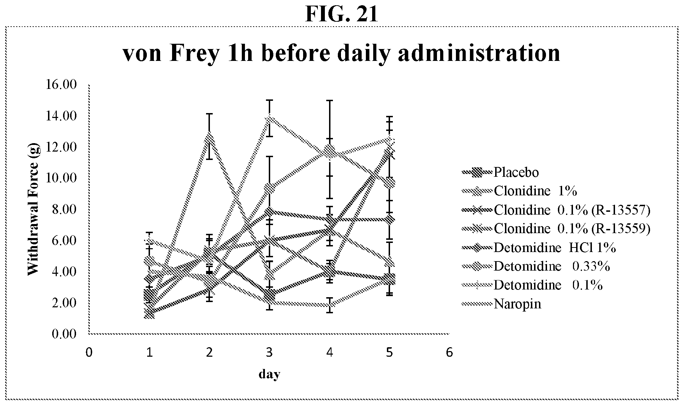

FIG. 19 shows the von Frey response with respect to each of the tested formulations as measured during the first few hours following topical application immediately after surgery. The scale is in hours following application of the formulations. FIG. 20 shows the von Frey response with respect to each of the tested formulations as measured one hour following each topical application over five days. FIG. 21 shows the von Frey response with respect to each of the tested formulations as measured 15 hours following the preceding evening's dose, and before the first dose of the current day, over five days.

## Claims

1. A topical formulation comprising:
0.001 (±10%) to 3 (±10%) wt% detomidine or a salt thereof;
a gelling agent; and,
a carrier that is suitable for topical administration to a human subject's skin, wherein the carrier comprises a water-miscible solubilizer that is present in an amount of 0.1-10% by weight of the formulation, and wherein said water-miscible solubilizer is chosen from sugar alcohols, diols, polyols, polyether alcohols, fatty acids, organic solvents, poloxamers, cyclodextrins; or waxes or oils; or a combination thereof;
wherein the formulation has a pH of 4.5 to 9, and,
wherein the formulation provides prolonged, substantially non-systemic treatment for pain.

2. The topical formulation according to claim 1, wherein topical administration of the formulation to a human subject is effective to induce formation of a depot of detomidine or a salt thereof in the human subject's skin, preferably in one or more of the subcutaneous layer, the epidermis, or the dermis.

3. The topical formulation according to any preceding claim comprising 0.008 to 3 wt% of said detomidine or salt thereof, preferably 0.01 to 2 wt% of said detomidine or salt thereof, more preferably 0.01 to 1.5 wt% of said detomidine or salt thereof, more preferably 0.033 to 1 wt% of said detomidine or salt thereof, and most preferably 0.033 to 0.33 wt% of said detomidine or salt thereof.

4. The topical formulation according to any preceding claim wherein said solubilizer comprises a diol or a polyol, preferably wherein said solubilizer comprises glycerol, propylene glycol, or a combination thereof.

5. The topical formulation according to any preceding claim wherein the carrier comprises a buffer that is effective to maintain the formulation at a pH of 5.2 (±10%) to 8.5 (±10%), preferably a pH of 5.5 (±10%) to 8.2 (±10%).

6. The topical formulation according to any preceding claim further comprising another therapeutic agent in addition to the detomidine or salt thereof, preferably wherein said other therapeutic agent comprises an analgesic.

7. The topical formulation according to any preceding claim further comprising one or more of a thickening agent, a preservative, a permeation enhancer, a wax, an emulsifying agent, an emollient, a humectant, a conditioning agent, antioxidant, and a viscosity regulator.

8. A topical formulation according to any preceding claim for use in the prolonged, non-systemic treatment for pain in a human subject in need thereof.

9. The topical formulation for use according to claim 8, wherein said pain is neuropathic pain, diabetic neuropathic pain, or postherpetic neuralgia.

10. The topical formulation for use according to claim 8, wherein the formulation is topically administered to the human subject on a once- or twice-daily basis.

11. A topical formulation according to any of claims 1-6 for use in forming a subcutaneous depot of detomidine or a salt thereof in a human subject, wherein the detomidine or salt thereof is released from said depot into said human subject in order to provide prolonged, non-systemic treatment of pain in the human subject, preferably wherein the depot releases a sufficient quantity of the detomidine or salt thereof to the patient such that administration of the formulation to the human subject on a once- or twice-daily basis is sufficient to provide the prolonged, non-systemic treatment of pain in the human subject.

12. The formulation according to claim 1 comprising up to 1% of said detomidine or salt thereof, producing a blood plasma concentration of no more than about 500 pg/mL of said detomidine or salt thereof in said human subject during the first 48 hours of twice-daily topical administration of said formulation.

13. The formulation according to claim 1, wherein administering said formulation once- or twice-daily topical to a human subject for up to four days results in a blood plasma concentration in said human subject that is less than that required to achieve a systemic therapeutic effect of the detomidine or salt thereof.

14. The topical formulation of claim 1, wherein the carrier comprises a buffer that is effective to maintain the formulation at pH 4.5 to 9; and wherein the formulation is effective to provide relief from pain in the human subject while producing a maximum blood plasma concentration of no more than about 1600 pg/mL after four days of twice daily administration of said formulation.

15. The topical formulation according to claim 14, wherein the solubilizer and buffer combine to dissolve the detomidine or salt thereof in said formulation at greater than 80%, greater than 85%, greater than 90%, or greater than 95% of the saturation point for the detomidine or salt thereof.

## Patentansprüche

1. Topische Formulierung, umfassend:
0,001 (±10 %) bis 3 (±10 %) Gew.-% Detomidin oder ein Salz davon,
ein Gelliermittel und
einen Träger, der sich für die topische Verabreichung an die Haut eines menschlichen Subjekts eignet, wobei der Träger einen wassermischbaren Lösungsvermittler umfasst, der in einer Menge von 0,1-10 Gew.-% der Formulierung vorliegt, und wobei der wassermischbare Lösungsvermittler
ausgewählt ist aus Zuckeralkoholen, Diolen, Polyolen, Polyetheralkoholen, Fettsäuren, organischen Lösungsmitteln, Poloxameren, Cyclodextrinen oder Wachsen oder Ölen oder einer Kombination davon,
wobei die Formulierung einen pH-Wert von 4,5 bis 9 aufweist und
wobei die Formulierung eine langfristige, im Wesentlichen nichtsystemische Schmerzbehandlung bereitstellt.

2. Topische Formulierung nach Anspruch 1, wobei durch die topische Verabreichung der Formulierung an einen Menschen die Bildung eines Depots von Detomidin oder eines Salzes davon in der Haut eines menschlichen Subjekts induziert wird, vorzugsweise in der subkutanen Schicht, der Epidermis und/oder der Dermis.

3. Topische Formulierung nach einem der vorhergehenden Ansprüche, umfassend 0,008 bis 3 Gew.-% an Detomidin oder dem Salz davon, vorzugsweise 0,01 bis 2 Gew.-% an Detomidin oder dem Salz davon, besonders bevorzugt 0,01 bis 1,5 Gew.-% an Detomidin oder dem Salz davon, ganz besonders bevorzugt 0,033 bis 1 Gew.-% an Detomidin oder dem Salz davon und am meisten bevorzugt 0,033 bis 0,33 Gew.-% an Detomidin oder dem Salz davon.

4. Topische Formulierung nach einem der vorhergehenden Ansprüche, wobei der Lösungsvermittler ein Diol oder ein Polyol umfasst, wobei der Lösungsvermittler vorzugsweise Glycerin, Propylenglykol oder eine Kombination davon umfasst.

5. Topische Formulierung nach einem der vorhergehenden Ansprüche, wobei der Träger einen Puffer umfasst, der die Formulierung auf einem pH-Wert von 5,2 (±10 %) bis 8,5 (±10 %), vorzugsweise einem pH-Wert von 5,5 (±10 %) bis 8,2 (±10 %), hält.

6. Topische Formulierung nach einem der vorhergehenden Ansprüche, weiterhin umfassen ein anderes Therapeutikum zusätzlich zu dem Detomidin oder dem Salz davon, wobei das andere Therapeutikum vorzugsweise ein Analgetikum umfasst.

7. Topische Formulierung nach einem der vorhergehenden Ansprüche, weiterhin umfassend ein Verdickungsmittel, einen Konservierungsstoff, einen Permeationsverstärker, ein Wachs, einen Emulgator, ein Emolliens, ein Feuchthaltemittel, einen Konditionierer, ein Antioxidationsmittel und/oder einen Viskositätsregulator.

8. Topische Formulierung nach einem der vorhergehenden Ansprüche zur Verwendung bei der langfristigen nichtsystemischen Schmerzbehandlung eines dessen bedürftigen menschlichen Subjekts.

9. Topische Formulierung zur Verwendung nach Anspruch 8, wobei es sich bei den Schmerzen um neuropathische Schmerzen, diabetische neuropathische Schmerzen oder postherpetische Neuralgie handelt.

10. Topische Formulierung zur Verwendung nach Anspruch 8, wobei die Formulierung dem menschlichen Subjekt auf ein- oder zweimal täglicher Basis topisch verabreicht wird.

11. Topische Formulierung nach einem der Ansprüche 1-6 zur Verwendung bei der Bildung eines subkutanen Depots von Detomidin oder einem Salz davon in einem menschlichen Subjekt, wobei das Detomidin oder das Salz davon aus dem Depot in das menschliche Subjekt freigesetzt wird, um eine langfristige nichtsystemische Schmerzbehandlung in dem menschlichen Subjekt bereitzustellen, wobei das Depot vorzugsweise eine ausreichende Menge an Detomidin oder dem Salz davon an den Patienten freisetzt, so dass die Verabreichung der Formulierung an das menschliche Subjekt auf ein- oder zweimal täglicher Basis zur Bereitstellung der langfristigen nichtsystemischen Schmerzbehandlung in dem menschlichen Subjekt ausreicht.

12. Formulierung nach Anspruch 1, umfassend bis zu 1 % an Detomidin oder dem Salz davon, die zu einer Blutplasmakonzentration von nicht mehr als etwa 500 pg/ml an Detomidin oder dem Salz davon in dem menschlichen Subjekt während der ersten 48 Stunden einer zweimal täglichen topischen Verabreichung der Formulierung führt.

13. Formulierung nach Anspruch 1, wobei die ein- oder zweimal tägliche topische Verabreichung der Formulierung an ein menschliches Subjekt über bis zu vier Tage zu einer Blutplasmakonzentration in dem menschlichen Subjekt führt, die geringer ist als die zum Erzielen einer systemischen therapeutischen Wirkung des Detomidin oder des Salzes davon erforderliche.

14. Topische Formulierung nach Anspruch 1, wobei der Träger einen Puffer umfasst, der die Formulierung auf einem pH-Wert von 4,5 bis 9 hält und wobei die Formulierung in dem menschlichen Subjekt für Schmerzlinderung sorgt und dabei eine maximale Blutplasmakonzentration von nicht mehr als etwa 1600 pg/ml nach vier Tagen einer zweimal täglichen Verabreichung der Formulierung erzeugt.

15. Topische Formulierung nach Anspruch 14, wobei Lösungsvermittler und Puffer zusammen das Detomidin oder das Salz davon in der Formulierung zu mehr als 80 %, mehr als 85 %, mehr als 90 % oder mehr als 95 % des Sättigungspunktes für das Detomidin bzw. das Salz davon lösen.

## Revendications

1. Formulation topique comprenant :
0,001 (± 10 %) à 3 (± 10 %) % en poids de détomidine ou d'un sel correspondant ;
un agent gélifiant ; et
un support qui est approprié pour une administration topique à la peau d'un sujet humain, le support comprenant un agent solubilisant miscible à l'eau qui est présent en une quantité de 0,1 à 10 % en poids de la formulation, et ledit agent solubilisant miscible à l'eau étant
choisi parmi des alcools de sucre, des diols, des polyols,
des alcools de polyéther, des acides gras, des solvants organiques, des poloxamères, des cyclodextrines ; ou des cires ou des huiles ; ou une combinaison correspondante ; la formulation ayant un pH de 4,5 à 9 et,
la formulation fournissant un traitement prolongé, sensiblement non systémique pour une douleur.

2. Formulation topique selon la revendication 1, une administration topique de la formulation à un sujet humain étant efficace pour induire une formation d'un dépôt de détomidine ou d'un sel correspondant dans la peau du sujet humain, préférablement dans un ou plusieurs parmi la couche sous-cutanée, l'épiderme ou le derme.

3. Formulation topique selon une quelconque revendication précédente comprenant 0,008 à 3 % en poids de ladite détomidine ou du sel correspondant, préférablement 0,01 à 2 % en poids de ladite détomidine ou du sel correspondant, plus préférablement 0,01 à 1,5 % en poids de ladite détomidine ou du sel correspondant, plus préférablement 0,033 à 1 % en poids de ladite détomidine ou du sel correspondant, et le plus préférablement 0,033 à 0,33 % en poids de ladite détomidine ou du sel correspondant.

4. Formulation topique selon une quelconque revendication précédente, ledit agent solubilisant comprenant un diol ou un polyol, préférablement ledit agent solubilisant comprenant du glycérol, du propylèneglycol ou une combinaison correspondante.

5. Formulation topique selon une quelconque revendication précédente, le support comprenant un tampon qui est efficace pour maintenir la formulation à un pH de 5,2 (± 10 %) à 8,5 (± 10 %), préférablement un pH de 5,5 (± 10 %) à 8,2 (± 10 %).

6. Formulation topique selon une quelconque revendication précédente comprenant en outre un autre agent thérapeutique en plus de la détomidine ou du sel correspondant, préférablement ledit autre agent thérapeutique comprenant un analgésique.

7. Formulation topique selon une quelconque revendication précédente comprenant en outre un ou plusieurs parmi un agent épaississant, un conservateur, un agent d'amélioration de la perméation, une cire, un agent émulsifiant, un émollient, un humectant, un agent de conditionnement, un antioxydant et un régulateur de viscosité.

8. Formulation topique selon une quelconque revendication précédente pour une utilisation dans le traitement prolongé, non systémique pour une douleur chez un sujet humain qui en a besoin.

9. Formulation topique pour une utilisation selon la revendication 8, ladite douleur étant une douleur neuropathique, une douleur neuropathique diabétique ou névralgie postherpétique.

10. Formulation topique pour une utilisation selon la revendication 8, la formulation étant administrée de manière topique au sujet humain sur une base quotidienne ou biquotidienne.

11. Formulation topique selon l'une quelconque des revendications 1 à 6 pour une utilisation dans la réalisation d'un dépôt sous-cutané de détomidine ou d'un sel correspondant chez un sujet humain, la détomidine ou le sel correspondant étant libéré(e) dudit dépôt dans ledit sujet humain afin de fournir un traitement prolongé, non systémique d'une douleur chez le sujet humain, préférablement, le dépôt libérant une quantité suffisante de la détomidine ou du sel correspondant au patient de sorte qu'une administration de la formulation au sujet humain sur une base quotidienne ou biquotidienne est suffisante pour fournir le traitement prolongé, non systémique d'une douleur chez le sujet humain.

12. Formulation selon la revendication 1 comprenant jusqu'à 1 % de ladite détomidine ou dudit sel correspondant, produisant une concentration dans le plasma sanguin de pas plus d'environ 500 pg/mL de ladite détomidine ou dudit sel correspondant chez ledit sujet humain pendant les premières 48 heures d'administration topique biquotidienne de ladite formulation.

13. Formulation selon la revendication 1, une administration de ladite formulation de manière topique quotidienne ou biquotidienne à un sujet humain pendant jusqu'à quatre jours entraînant une concentration dans le plasma sanguin chez ledit sujet humain qui est inférieure à celle requise pour atteindre un effet thérapeutique systémique de la détomidine ou du sel correspondant.

14. Formulation topique selon la revendication 1, le support comprenant un tampon qui est efficace pour maintenir la formulation à pH 4,5 à 9 ; et la formulation étant efficace pour fournir un soulagement de la douleur chez un sujet humain tout en produisant une concentration maximale dans le plasma sanguin de pas plus d'environ 1600 pg/mL après quatre jours d'administration biquotidienne de ladite formulation.

15. Formulation topique selon la revendication 14, l'agent solubilisant et le tampon se combinant pour dissoudre la détomidine ou le sel correspondant dans ladite formulation à raison de plus de 80 %, plus de 85 %, plus de 90 % ou plus de 95 % du point de saturation pour la détomidine ou le sel correspondant.
